# EUROPÄISCHE PATENTSCHRIFT

(11) **EP 0 590 555 B1**
(45) Veröffentlichungstag und Bekanntmachung des Hinweises auf die Patenterteilung: **24.01.1996**
(21) Anmeldenummer: 93115520.4
(22) Anmeldetag: 25.09.1993
(51) Int. Cl.: C07C 323/44, C07C 317/28, C07C 273/18

(54) **Verfahren zur Herstellung von aromatischen Harnstoffen, die eine Thioeter-oder Sulfonylgruppe aufweisen**
Process for the preparation of aromatic ureas, containing a thioether or a sulphonyl group
Procédé pour la préparation d'urées aromatiques renfermant un groupement thioéther ou sulfonyle

(30) Priorität: 01.10.1992 DE 4233033
(43) Veröffentlichungstag der Anmeldung: 06.04.1994
(73) Patentinhaber: BASF Aktiengesellschaft, D-67063 Ludwigshafen (DE)
(72) Erfinder: Siegel, Bernd, Dr., D-6700 Ludwigshafen (DE); Patsch, Manfred, Dr., D-6706 Wachenheim (DE)

(56) Entgegenhaltungen:
- EP-A- 0 503 385
- EP-A- 0 531 689
- DE-B- 2 256 275

## Beschreibung

Die vorliegende Erfindung betrifft ein neues Verfahren zur Herstellung von N-Phenyl- oder N-Naphthylharnstoffen, die über eine Thioether- oder Sulfonylgruppe im Molekül verfügen, wobei man einen unsubstituierten N-Phenyl- oder N-Naphthylharnstoff mit einem Amin, das eine Thioether- oder Sulfonylgruppe im Molekül aufweist, zur Reaktion bringt.

In der US-A-5 091 016 sind aromatische Harnstoffe, die eine Thioether- oder Sulfonylgruppe aufweisen, beschrieben. Ihre Herstellung ist jedoch umständlich, da sie über aromatische Isocyanate abläuft.

Ein Verfahren zur Herstellung von Harnstoffen ist auch in EP-A-531689 beschrieben; die Zielprodukte besitzen aber keine Thioether- oder Sulfonylgruppe und das Verfahren wird abgebrochen bevor Nebenprodukte entstehen.

Aufgabe der vorliegenden Erfindung war es nun, ein neues Verfahren zur Herstellung von aromatischen Harnstoffen, die eine Thioether- oder Sulfonylgruppe im Molekül aufweisen, bereitzustellen. Das neue Verfahren sollte auf einfache Weise durchführbar sein und die Zielprodukte in guter Ausbeute und hoher Reinheit liefern. Außerdem sollten die zur Anwendung gelangenden Edukte auf einfache Weise zugänglich sein.

Es wurde gefunden, daß die Herstellung von Harnstoffen der Formel I 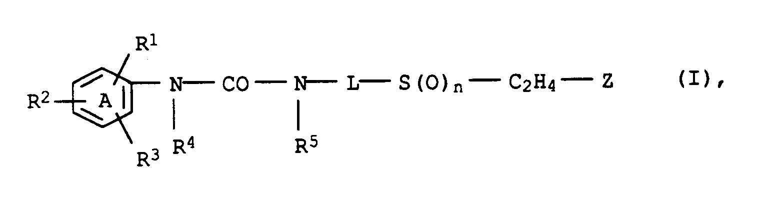
in der
- n: 0 oder 2,
- R¹,R² und R³: unabhängig voneinander jeweils Wasserstoff, C₁-C₄-Alkyl, C₁-C₄-Alkoxy, Halogen, Hydroxy, Hydroxysulfonyl, Carboxyl, Cyano, Nitro, C₂-C₄-Alkanoylamino oder C₁-C₄-Alkoxycarbonylamino
- R⁴ und R⁵: unabhängig voneinander jeweils Wasserstoff, C₁-C₄-Alkyl oder Phenyl,
- L: C₂-C₄-Alkylen, das gegebenenfalls einmal durch Sauerstoff, Imino oder N-(C₁-C₄-Alkyl)imino unterbrochen ist, oder NR⁵ und L zusammen den Rest der Formel 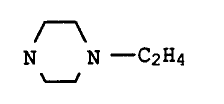 und
- Z: Hydroxy oder eine unter alkalischen Reaktionsbedingungen abspaltbare Gruppe bedeuten und
der Ring A benzoanelliert sein kann, vorteilhaft gelingt, wenn man einen Phenylharnstoff der Formel II 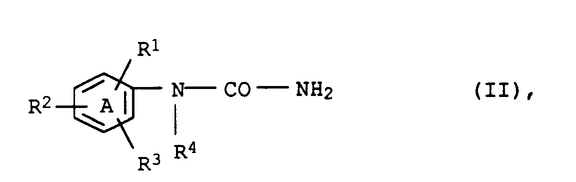
in der R¹, R², R³, R⁴ und der Ring A jeweils die obengenannte Bedeutung besitzen, mit einem Amin der Formel III 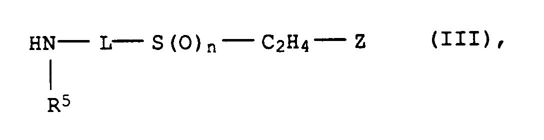
in der n, R⁵, L und Z jeweils die obengenannte Bedeutung besitzen, bei einer Temperatur von 80 bis 180°C gegebenenfalls in Gegenwart eines inerten Verdünnungsmittels umsetzt.

Alle in den obengenannten Formeln auftretenden Alkyl- und Alkylengruppen können sowohl geradkettig als auch verzweigt sein.

Reste R¹, R², R³, R⁴ und R⁵ sind z.B. Methyl, Ethyl, Propyl, Isopropyl, Butyl, Isobutyl oder sec-Butyl.

Reste R¹, R² und R³ sind weiterhin z.B. Methoxy, Ethoxy, Propoxy, Isopropoxy, Butoxy, Isobutoxy, sec-Butoxy, Fluor, Chlor, Brom, Acetylamino, Propionylamino, Butylrylamino, Isobutyrylamino, Methoxycarbonylamino, Ethoxycarbonylamino, Propoxycarbonylamino, Isopropoxycarbonylamino oder Butoxycarbonylamino.

Reste L sind z.B. Ethylen, 1,2- oder 1,3-Propylen, 1,2-, 1,3-, 1,4- oder 2,3-Butylen, 3-Oxa-1,4-butylen, 3-Aza-1,4-butylen, 3-Aza-3-methyl-1,4-butylen oder 3-Aza-3-ethyl-1,4-butylen.

Der Rest Z steht u.a. für eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, gegebenenfalls substituiertes Phenylsulfonyloxy, C₁-C₄-Alkanoyloxy, C₁-C₄-Dialkylamino oder ein Rest der Formel 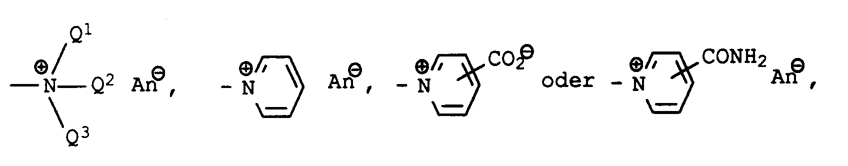
wobei Q¹, Q² und Q³ unabhängig voneinander jeweils die Bedeutung von C₁-C₄-Alkyl oder Benzyl und An^{⊖} jeweils die Bedeutung eines Äquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommmen.

Geeignete inerte Verdünnungsmittel, die im erfindungsgemäßen Verfahren zur Anwendung gelangen können, sind z.B. Wasser oder organische Lösungsmittel, wie Toluol, Xylol, Nitrobenzol, Chlorbenzol, Dichlorbenzol, N,N-Dimethylformamid, N,N-Dimethylacetamid oder N-Methylpyrrolidinon.

Das neue Verfahren wird bei einer Temperatur von 80 bis 180°C, vorzugsweise 95 bis 150°C, durchgeführt, wobei man in der Regel unter atmosphärischem Druck arbeitet.

Je Mol Phenylharnstoff der Formel II kommen im allgemeinen 1 bis 1,5 mol, vorzugsweise 1 bis 1,2 mol, Amin der Formel III zur Anwendung.

Bevorzugt ist eine Verfahrensweise, in der man die Umsetzung in Gegenwart eines inerten Verdünnungsmittels durchführt. In diesem Fall kommen üblicherweise 100 bis 500 Gew.-%, vorzugsweise 150 bis 300 Gew.-%, jeweils bezogen auf das Gewicht an Phenylharnstoff II, Verdünnungsmittel zur Anwendung.

Besonders geeignete Verdünnungsmittel sind z.B. Toluol, Xylol oder N,N-Dimethylacetamid.

Bevorzugt ist ein Verfahren zur Herstellung von Harnstoffen der Formel I, in der der Ring A nicht benzoanelliert ist.

Bevorzugt ist weiterhin ein Verfahren zur Herstellung von Harnstoffen der Formel I, in der R³ Wasserstoff bedeutet.

Bevorzugt ist weiterhin ein Verfahren zur Herstellung von Harnstoffen der Formel I, in der R¹ Nitro, Acetylamino oder Methoxycarbonylamino und R² Wasserstoff, C₁-C₄-Alkyl, C₁-C₄-Alkoxy, Chlor Hydroxysulfonyl oder Carboxyl bedeuten.

Bevorzugt ist weiterhin ein Verfahren zur Herstellung von Harnstoffen der Formel I, in der R⁴ Wasserstoff und R⁵ Wasserstoff oder Methyl bedeuten.

Bevorzugt ist weiterhin ein Verfahren zur Herstellung von Harnstoffen der Formel I, in der L C₂-C₄-Alkylen, das gegebenenfalls einmal durch Sauerstoff unterbrochen ist, bedeutet.

Bevorzugt ist weiterhin ein Verfahren zur Herstellung von Harnstoffen der Formel I, in der Z Hydroxy bedeutet.

Hervorzuheben ist eine Verfahrensweise zur Herstellung von Harnstoffen der Formel Ia 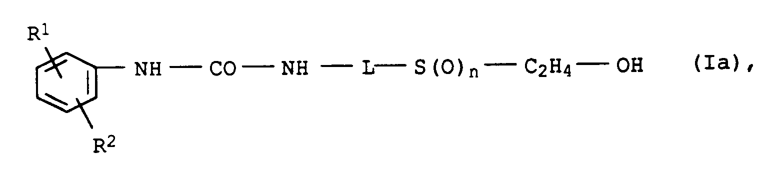
in der
- n: 0 oder 2,
- R¹: Nitro oder Acetylamino,
- R²: Wasserstoff, Methyl, Methoxy, Chlor, Hydroxysulfonyl oder Carboxyl und
- L: C₂-C₄-Alkylen, das gegebenenfalls einmal durch Sauerstoff unterbrochen ist, bedeuten,
wobei man einen Phenylharnstoff der Formel IIa 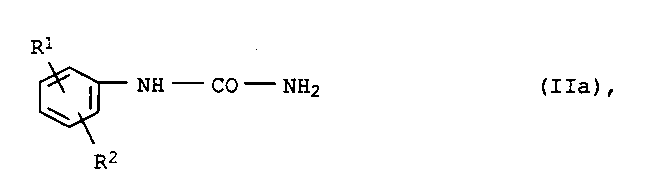
in der R¹ und R² jeweils die obengenannte Bedeutung besitzen, mit einem Amin der Formel IIIa

H₂N―L―S(O)ₙ―C₂H₄―OH (IIIa),

in der n und L jeweils die obengenannte Bedeutung besitzen, umsetzt.

Das erfindungsgemäße Verfahren wird zweckmäßig so durchgeführt, daß man Phenylharnstoff II, Amin III und gegebenenfalls das inerte Verdünnungsmittel vorlegt und unter Rühren auf die erfindungsgemäße Temperatur erhitzt.

Nach einer Reaktionsdauer, die in der Regel 2 bis 40 Stunden beträgt, wird das Reaktionsgemisch abgekühlt und nach an sich bekannten Methoden aufgearbeitet. Beispielsweise kann man das gegebenenfalls vorhandene Verdünnungsmittel abdestillieren oder aber wenn es sich um ein organisches, wassermischbares Verdünnungsmittel handelt, das Reaktionsgemisch mit Wasser verdünnen, das als Niederschlag anfallende Zielprodukt abtrennen und gegebenenfalls waschen und trocknen.

Abhängig von der Bedeutung von n und Z können verschiedene Herstellvarianten durchgeführt werden. Beispielsweise kann man zunächst ein Amin III, das eine Thioethergruppe aufweist mit dem Phenylharnstoff II zur Reaktion bringen und gegebenenfalls anschließend eine Oxidation zur Sulfonylgruppe, beispielsweise mit Wasserstoffperoxid, vornehmen. Es ist aber auch möglich, ein Amin III, das bereits eine Sulfonylgruppe im Molekül aufweist, mit dem Phenylharnstoff II umzusetzen.

Außerdem kann man ein Amin III zur Reaktion bringen, das über eine Hydroxylethylgruppe verfügt, und die unter alkalischen Reaktionsbedingungen abspaltbare Gruppe gegebenenfalls anschließend einführen. Es ist aber auch möglich, ein Amin III, das bereits eine unter alkalischen Reaktionsbedingungen abspaltbare Gruppe im Molekül aufweist, direkt mit dem Phenylharnstoff II umzusetzen. In beiden Fällen kann wie oben ausgeführt, das Amin III entweder über eine Thioether- oder Sulfonylgruppe verfügen.

Bei den Phenylharnstoffen der Formel II und den Aminen der Formel III handelt es sich im allgemeinen um an sich bekannte Verbindungen. Phenylharnstoffe der Formel II sind z.B. aus Houben-Weyl "Methoden der Organischen Chemie", Band 8, Seiten 149 bis 163, bekannt oder können nach den dort beschriebenen Methoden erhalten werden. Amine der Formel III sind z.B. in der JP-A-24900/1969 beschrieben oder können z.B. aus Ethylenimin und 2-Mercaptoethanol nach an sich bekannten Methoden, wie in Houben-Weyl "Methoden der Organischen Chemie", Band 9, Seite 135, genannt, erhalten werden.

Mittels des erfindungsgemäßen Verfahrens, das sowohl in kontinuierlicher als auch diskontinuierlicher Arbeitsweise vorgenommen werden kann, werden die Zielprodukte auf einfache Weise und in guter Ausbeute und hoher Reinheit erhalten.

Die mittels des erfindungsgemäßen Verfahrens erhältlichen Harnstoffe der Formel I sind wertvolle Zwischenprodukte für die Herstellung von Reaktivfarbstoffen, wie sie beispielsweise in der US-A-4 841 028, US-A-5 091 016, EP-A- 503 385 oder in der älteren Patentanmeldung EP-A-515 844 beschrieben sind.

Die folgenden Beispiele sollen die Erfindung näher erläutern.

### Beispiel 1

a) 770 g eines wasserfeuchten Nutschgutes (Trockengehalt: 47 Gew.-%) von 3-Nitrophenylharnstoff wurden mit 860 g Xylol (Isomerengemisch) und 290,4 g 2-Aminoethyl-2'-hydroxyethyl-sulfid versetzt und zum Sieden erhitzt. Das bei 95°C siedende Xylol-Wasser-Azeotrop wurde abdestilliert und anschließend das Reaktionsgemisch zur Entfernung des restlichen Xylols auf 140°C erhitzt. Nach Beendigung der Umsetzung (DC-Kontrolle) verblieben 495 g der Verbindung der Formel 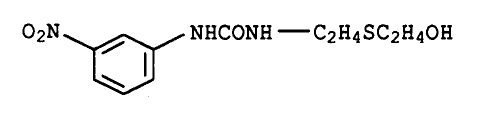
   - ¹H-NMR(d⁶-DMSO):: δ = 2,55(m, 4H,CH₂-S-CH₂); 3,30 (q, 2H, CH₂); 3,53 (q, 2H, CH₂); 4,82 (bs, 1H, OH); 6,50(t,1H, NH); 7,50-7,75(m,3H, Aromaten-H); 8,53 (s,1H, Aromaten-H); 9,22 (s, 1H, NH)ppm.
b) 542 g der unter a) beschriebenen Verbindung wurden mit 2 g Wolframsäure und 1600 ml Wasser (Temp.:80°C) versetzt und mit 565 g 30 gew.-%igem wäßrigem Wasserstoffperoxid bei einer Temperatur von 90 bis 95°C oxidiert. Nach Beendigung der Peroxidzugabe wurde das Reaktionsgemisch bis zur Kristallisation bei 90°C getempert. Anschließend wurde auf Raumtemperatur abgekühlt und der entstandene Niederschlag abgesaugt. Es verblieben 586 g eines gelb gefärbten Feststoffes der Formel 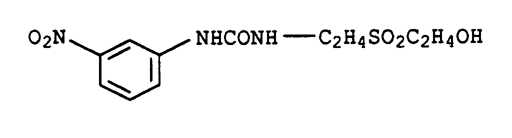
Analog Beispiel 1 werden die in der folgenden Tabelle aufgeführten Verbindungen erhalten.

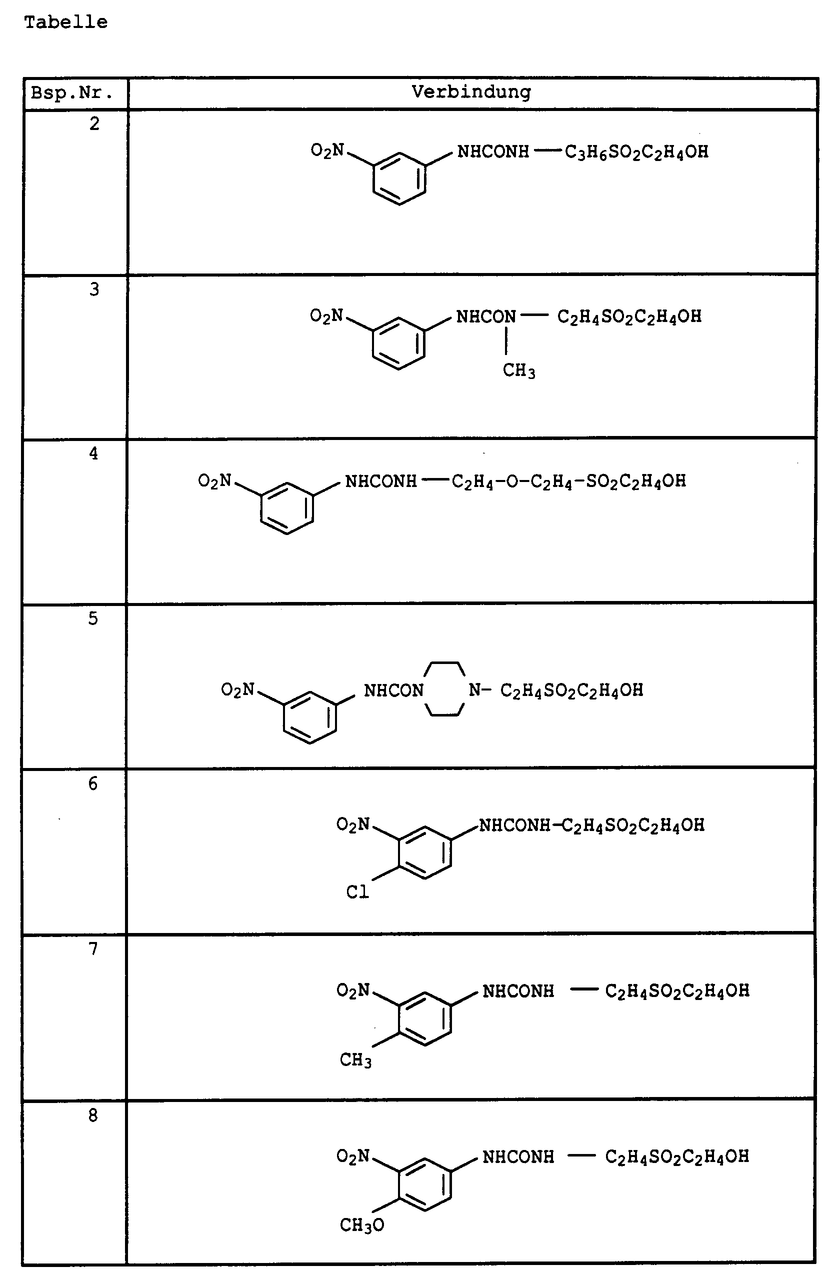

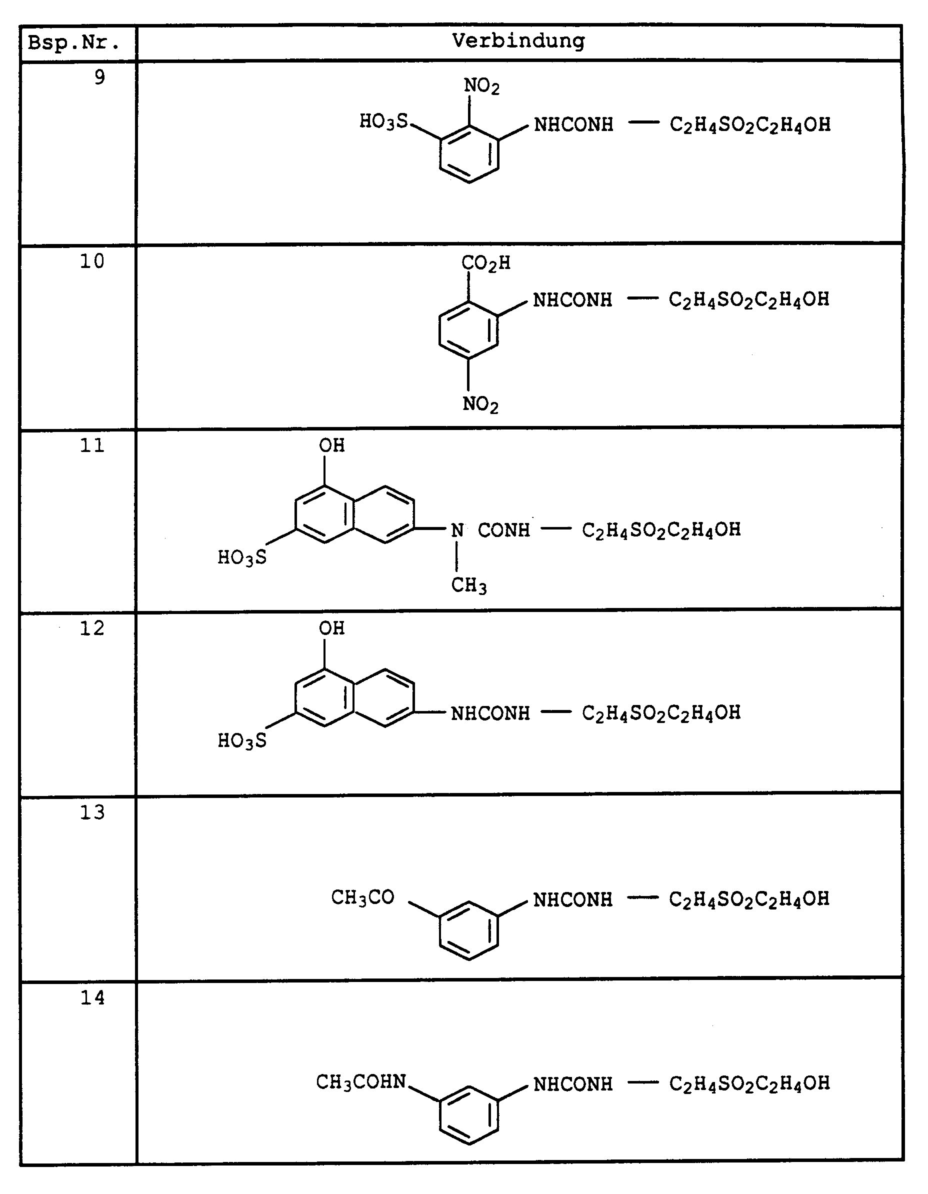

### Beispiel 15

Zu einem Gemisch aus 500 g Eis und 56,3 g konz. Schwefelsäure wurden 121 g 2-Aminoethyl-2'-hydroxyethylsulfid und 1,0 g Natriumwolframat x 2 H₂O gegeben und auf 80°C erwärmt. Der Thioether wurde bei obiger Temperatur mit 227 g 30 gew.-%igem wäßrigem Wasserstoffperoxid zum Sulfon oxidiert. Nach Beendigung der Reaktion wurde das Reaktionsgemisch auf Raumtemperatur abgekühlt, mit 25 gew.%iger Natronlauge ein pH-Wert von 7 eingestellt und mit 385 g wasserfeuchtem Nutschguts (Trockengehalt: 44 Gew.-%) von 3-Nitrophenylharnstoff versetzt. Nach Zugabe von 300 ml N,N-Dimethylacetamid wurde das Reaktionsgemisch zum Sieden erhitzt und das Wasser weitestgehend abdestilliert. Zur vollständigen Umsetzung wurde das Reaktionsgemisch im Anschluß noch 4 Stunden bei 140°C getempert. Nach Beendigung der Reaktion wurde auf Raumtemperatur abgekühlt und das Reaktionsprodukt durch Zugabe von 1500 ml Wasser ausgefällt, abgesaugt, gewaschen und getrocknet. Es verblieben 195 g der in Beispiel 1b beschriebenen Verbindung.

Die in der Tabelle angeführten Verbindungen sind ebenfalls nach dem in Beispiel 15 beschriebenen Weg erhältlich.

### Beispiel 16

285 g der Verbindung der Formel 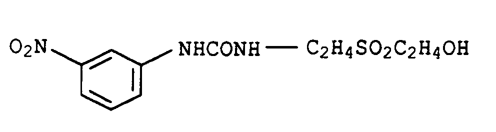
wurden mit 408 g Acetanhydrid auf 95°C erhitzt. Nach beendeter Umsetzung (DC-Kontrolle) wurde das überschüssige Acylierungsmittel mit Wasser zerstört und der sich bildende Niederschlag bei Raumtemperatur abgesaugt. Es wurden 323 g der Verbindung der Formel 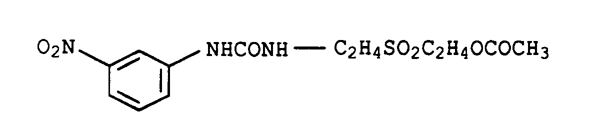
erhalten. Schmp.: 161-163°C

In analoger Weise können die folgenden Derivate hergestellt werden:

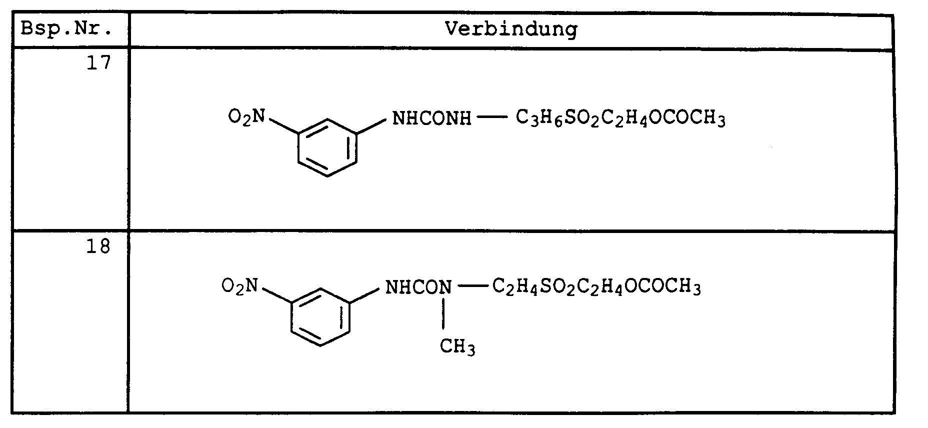

## Patentansprüche

1. Verfahren zur Herstellung von Harnstoffen der Formel I 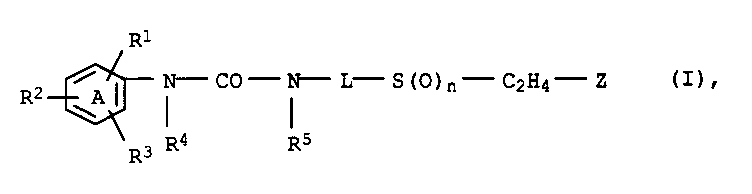 in der
n 0 oder 2,
R¹,R² und R³ unabhängig voneinander jeweils Wasserstoff, C₁-C₄-Alkyl, C₁-C₄-Alkoxy, Halogen, Hydroxy, Hydroxysulfonyl, Carboxyl, Cyano, Nitro, C₂-C₄-Alkanoylamino oder C₁-C₄-Alkoxycarbonylamino
R⁴ und R⁵ unabhängig voneinander jeweils Wasserstoff, C₁-C₄-Alkyl oder Phenyl,
L C₂-C₄-Alkylen, das gegebenenfalls einmal durch Sauerstoff, Imino oder N-(C₁-C₄-Alkyl)imino unterbrochen ist, oder NR⁵ und L zusammen den Rest der Formel 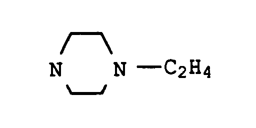 und
Z Hydroxy oder eine unter alkalischen Reaktionsbedingungen abspaltbare Gruppe bedeuten und
der Ring A benzoanelliert sein kann,
dadurch gekennzeichnet, daß man einen Phenylharnstoff der Formel II 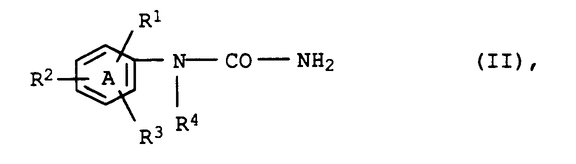 in der R¹, R², R³, R⁴ und der Ring A jeweils die obengenannte Bedeutung besitzen, mit einem Amin der Formel III 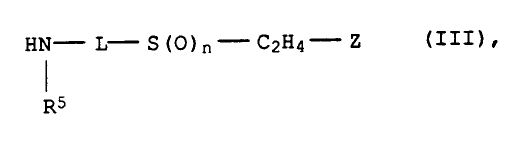 in der n, R⁵, L und Z jeweils die obengenannte Bedeutung besitzen, bei einer Temperatur von 80 bis 180°C gegebenenfalls in Gegenwart eines inerten Verdünnungsmittels umsetzt.

2. Verfahren nach Anspruch 1, dadurch gekennzeichnet, daß man die Umsetzung in Gegenwart eines inerten Verdünnungsmittels durchführt.

3. Verfahren nach Anspruch 1, dadurch gekennzeichnet, daß man die Umsetzung bei einer Temperatur von 95 bis 150°C durchführt.

## Claims

1. A process for preparing ureas of the formula I 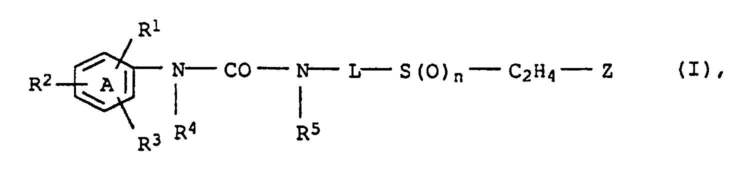 where
n is 0 or 2,
R¹, R² and R³ independently of one another are each hydrogen, C₁-C₄-alkyl, C₁-C₄-alkoxy, halogen, hydroxyl, hydroxysulfonyl, carboxyl, cyano, nitro, C₂-C₄-alkanoylamino or C₁-C₄-alkoxycarbonylamino,
R⁴ and R⁵ independently of one another are each hydrogen, C₁-C₄-alkyl or phenyl,
L is C₂-C₄-alkylene which may be interrupted once by oxygen, imino or N-(C₁-C₄-alkyl)imino, or NR⁵ and L together are the radical of the formula 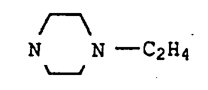 and
Z is hydroxyl or a group which can be eliminated under alkaline reaction conditions and
the ring A can be benzo-fused,
which comprises reacting a phenylurea of the formula II 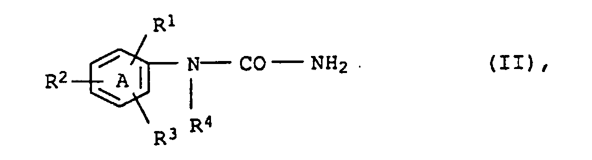 where R¹, R², R³, R⁴ and the ring A each have the above-mentioned meanings, with an amine of the formula III 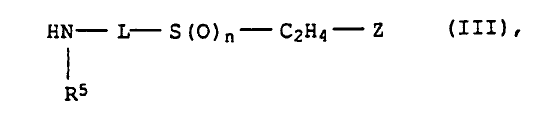 where n, R⁵, L and Z each have the abovementioned meanings, at from 80 to 180°C, in the presence or absence of an inert diluent.

2. A process as claimed in claim 1, wherein the reaction is carried out in the presence of an inert diluent.

3. A process as claimed in claim 1, wherein the reaction is carried out at from 95 to 150°C.

## Revendications

1. Procédé de préparation d'urées de la formule I 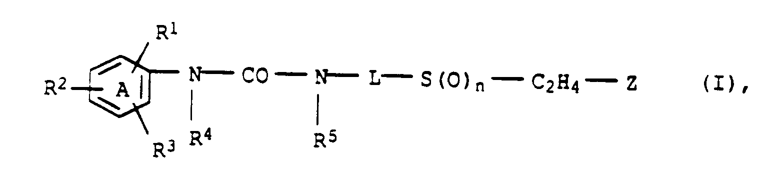 dans laquelle
n est égal à 0 ou à 2,
R¹, R² et R³ représentent, chacun indépendamment l'un de l'autre, un atome d'hydrogène, un radical alkyle en C₁ à C₄, alcoxy en C₁ à C₄, un atome d'halogène, un radical hydroxyle, hydroxysulfonyle, carboxyle, cyano, nitro, alcanoylamino en C₂ à C₄, ou alcoxycarbonylamino en C₁ à C₄,
R⁴ et R⁵ représentent, chacun indépendamment l'un de l'autre, un atome d'hydrogène, un radical alkyle en C₁ à C₄ ou phényle,
L représente un groupe alkylène en C₂ à C₄, qui est éventuellement une fois interrompu par un atome d'oxygène, un radical imino ou N-(alkyle en C₁ à C₄)imino, ou bien le groupe NR⁵ et L représentent ensemble le reste de la formule et 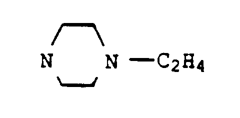 et
Z représente le radical hydroxyle ou un radical susceptible de se séparer dans des conditions réactionnelles alcalines et
le noyau A peut être benzocondensé,
caractérisé en ce que l'on fait réagir une phénylurée de la formule II 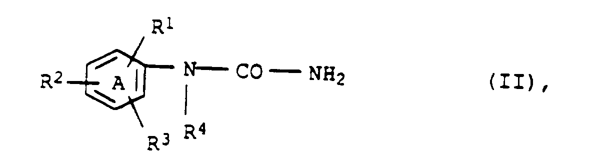 dans laquelle R¹, R², R³, R⁴ et le noyau A possèdent chacun les significations qui leur ont été attribuées ci-dessus, avec une amine de la formule III 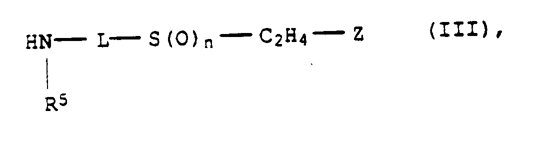 dans laquelle n, R⁵, L et Z possèdent chacun les significations qui leur ont été attribuées ci-dessus, à une température de 80°C à 180°C, éventuellement en présence d'un diluant inerte.

2. Procédé suivant la revendication 1, caractérisé en ce que l'on entreprend la réaction en présence d'un diluant inerte.

3. Procédé suivant la revendication 1, caractérisé en ce que l'on entreprend la réaction à une température de 95 à 150°C.
